# EUROPEAN PATENT APPLICATION

(11) **EP 4 748 435 A1**
(43) Date of publication of application: **27.05.2026**
(21) Application number: 24843050.6
(22) Date of filing: 11.07.2024
(51) Int. Cl.: A61N 1/04, A61B 5/263, A61B 5/268

(54) **ELECTRODE FOR LIVING BODIES AND METHOD FOR MANUFACTURING SAME**

(30) Priority: 20.07.2023 JP 2023118579
(71) Applicant: NOK Corporation, Minato-ku Tokyo 105-8585 (JP)
(72) Inventor: HAYASHI, Taisei, Fujisawa-shi, Kanagawa 251-0042 (JP); UEDA, Keisuke, Fujisawa-shi, Kanagawa 251-0042 (JP)
(74) Representative: TBK
(86) International application number: PCT/JP2024/025097
(87) International publication number: WO 2025/018259

(57) **Abstract**

A bioelectrode includes a porous substrate having a plurality of pores formed inside; and a conductive coating material coating a surface of the porous substrate and inner surfaces of the plurality of pores.

## Description

### TECHNICAL FIELD

The present invention relates to bioelectrodes that contact a surface of a living body.

### BACKGROUND ART

Various devices, such as electrical muscle stimulation (EMS) devices, low-frequency therapeutic devices, and biometric devices include a bioelectrode that contacts a surface of a living body. For example, Patent Document 1 discloses a bioelectrode made of a resin containing admixed conductive fillers. Patent Document 2 discloses a bioelectrode having a structure in which a conductor formed of PEDOT-PSS is attached to a substrate.

### Related Art Documents

### Patent Documents

Patent Document 1 Japanese Patent Application Laid-Open Publication No. 2001-292972
Patent Document 2 International Publication No. 2013/073673

### SUMMARY OF THE INVENTION

### Problem to be Solved by the Invention

The bioelectrode formed of a resin containing admixed conductive fillers disclosed in Patent Document 1 tends to lack flexibility. Consequently, the bioelectrode is unable to sufficiently deform to align with a surface of a living body, which results in difficulty in reducing a contact impedance between the bioelectrode and the surface of the living body. Further, the conductor attached to the surface of a substrate as disclosed in Patent Document 2 is prone to defects such as breakage or peeling of the conductor under deformation (for example, expansion and contraction) of the substrate. In view of the above circumstances, an object of one aspect of the present disclosure is to provide a bioelectrode that has both flexibility and conductivity.

### Means of Solving the Problems

To solve the above problems, a bioelectrode according to an aspect of the present disclosure includes a porous substrate having a plurality of pores formed inside; and a conductive coating material coating a surface of the porous substrate and inner surfaces of the plurality of pores.

A method for manufacturing a bioelectrode according to an aspect of the present disclosure includes a forming step of forming a porous substrate having a plurality of pores formed inside; and a coating step of coating a surface of the porous substrate and inner surfaces of the plurality of pores with a conductive coating material.

### BRIEF DESCRIPTION OF THE DRAWINGS

[Fig. 1] Fig. 1 is a cross-sectional view of a bioelectrode according to an embodiment.
[Fig. 2] Fig. 2 is a diagram illustrating effects of the embodiment.
[Fig. 3] Fig. 3 is a flowchart illustrating a method of manufacturing a bioelectrode.

### MODES FOR CARRYING OUT THE INVENTION

An embodiment of the present disclosure will now be described with reference to the drawings. In the drawings, dimensions and scales of each element may differ from those of actual products. The following description of the embodiment is a description of one exemplary implementation of the present disclosure. Therefore, the scope of the present disclosure is not limited to the embodiment illustrated in the following.

### A: Configuration of Bioelectrode 100

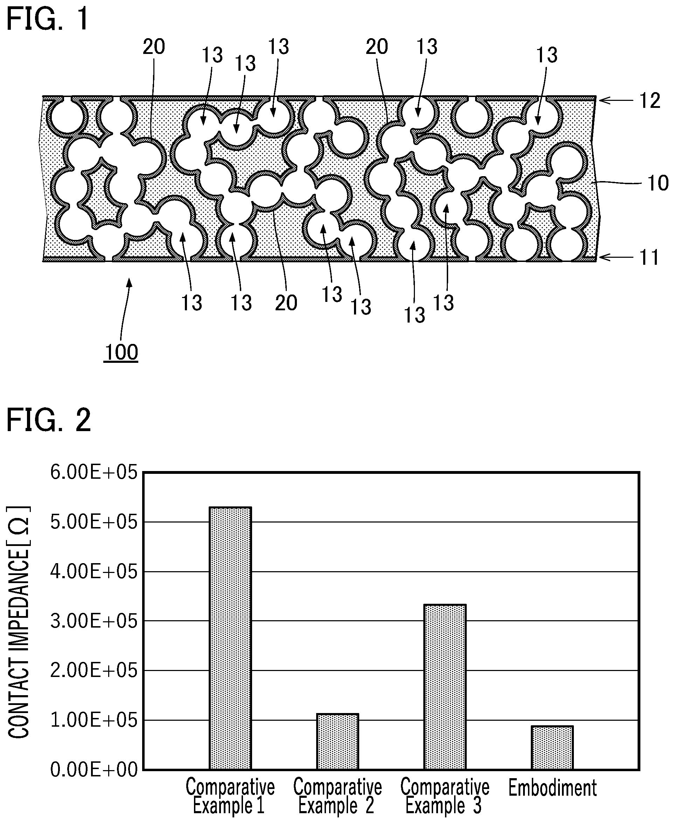

Fig. 1 is a cross-sectional view of a bioelectrode 100 according to one embodiment of the present disclosure. The bioelectrode 100 of the present embodiment is an electrode device that contacts the surface of a living body (i.e., skin). The bioelectrode 100 is used in an EMS device or a low-frequency therapeutic device that applies a stimulus to a living body upon supply of an electric current, for example. The bioelectrode 100 is also used in a biometric device that measures biological information. The biometric device is, for example, an electrocardiograph that detects an electrocardiogram of a living body, an electromyograph that detects an electromyogram of a living body, or an electroencephalograph that detects an electroencephalogram of a living body. As illustrated in Fig. 1, the bioelectrode 100 includes a porous substrate 10 and a coating material 20.

The porous substrate 10 is a flat plate-shaped body, which is formed to have a predetermined thickness. The surface of the porous substrate 10 includes a first surface 11 and a second surface 12. The first surface 11 faces the surface of the living body. The second surface 12 opposes the first surface 11. The shape of the porous substrate 10 is formed as a freely selected planar shape, such as a rectangular shape or a circular shape.

A plurality of pores 13 (voids) is formed in the porous substrate 10. Each of the plurality of pores 13 is a spherical bubble having a diameter sufficiently smaller than the plate thickness of the porous substrate 10. The porous substrate 10 of the present embodiment has a continuous pore structure in which each of the plurality of pores 13 communicates between the first surface 11 and the second surface 12. That is, each of the plurality of pores 13 extends through an entire range from the first surface 11 to the second surface 12. The plurality of pores 13 opens to each of the first surface 11 and the second surface 12, and a space facing the first surface 11 and a space facing the second surface 12 communicate with each other via the plurality of pores 13. It is of note that the plurality of pores 13 of the porous substrate 10 includes individual pores 13 that do not communicate with other of the plurality of pores 13.

The porous substrate 10 is a flexible substrate formed of an insulating resin. The porous substrate 10 is elastically deformable with flexibility so as to conform to a shape of a surface of a living body. The porous substrate 10 is formed of a rubber material, such as polyurethane rubber (PUR) or polyolefin rubber (PO), silicone rubber (SR), ethylene propylene diene rubber (EPDM), or fluorosilicone rubber (FVMQ), for example. The porous substrate 10 of the present embodiment does not include a conductive material such as a conductive filler.

The coating material 20 is a conductive film (coating) that is formed on the porous substrate 10. Specifically, the coating material 20 coats the first surface 11 and the second surface 12 of the porous substrate 10, and coats inner surfaces of the plurality of pores 13. That is, the coating material 20 is formed not only on the outer surface of the porous substrate 10 but also inside the porous substrate 10. Portions of the coating material 20 that coat the first surface 11 and the second surface 12 are respectively formed as a thin film with a predetermined thickness in a planar shape.

The film thickness of the coating material 20 is sufficiently smaller than the radius of each of the plurality of pores 13. Therefore, a space is formed inside the coating material 20 that coats an inner surface of each of the plurality of pores 13. That is, the coating material 20 inside each of the plurality of pores 13 is formed to have a spherical shell shape. The coating material 20 is formed to be continuous across two or more of the plurality of pores 13 communicating between the first surface 11 and the second surface 12. Therefore, a portion (first portion) of the coating material 20 coating the first surface 11 and a portion (second portion) coating the second surface 12 are electrically connected to each other via a portion (third portion) coating the inner surface of each of the plurality of pores 13.

The coating material 20 is formed of a conductive polymer (ICP: Intrinsically Conducting Polymer). Specifically, the coating material 20 contains PEDOT (poly(3,4-ethylenedioxythiophene)), which is an exemplary conductive polymer. For example, the coating material 20 is formed of a mixture of PEDOT and PSS (polystyrene sulfonate). As described above, in the present embodiment, the coating material 20 is formed of a conductive polymer. Accordingly, the coating material 20 can be easily formed on the inner surface of each of the plurality of pores 13 in the porous substrate 10. It is of note that the material of the coating material 20 is not limited to the above example.

As described above, the present embodiment uses the porous substrate 10 formed with the plurality of pores 13 inside. Therefore, flexibility is easily ensured as compared with a bioelectrode formed of, for example, a resin material in which conductive fillers are admixed. That is, in the present embodiment, since the bioelectrode 100 sufficiently deforms to conform to the surface of the living body, the bioelectrode 100 remains in close and stable contact with the surface. Therefore, contact impedance between the bioelectrode 100 and the surface of the living body can be reduced.

Fig. 2 shows measurement results of the contact impedance. Fig. 2 shows the measurement results of the embodiment in which the conductive polymer coating material 20 is formed on the porous substrate 10, and the measurement results of more than one Comparative Examples (Comparative Examples 1 to 3).

For measurement of the contact impedance, an LCR meter (model number: ZM2371) manufactured by NF Corporation was used. Specifically, a bioelectrode 100 having dimensions of 15mm×15mm×2mm was used as a measurement target, and the contact impedance was measured with a 1 Vrms (rms value) voltage applied at a frequency of 10Hz.

In Comparative Example 1, an electrode is formed on a surface of a cloth substrate. As will be apparent from Fig. 2, the contact impedance in Comparative Example 1 is sufficiently large, making it difficult to be used alone in practice. In Comparative Example 2, an electrode was formed in the same manner as in Comparative Example 1, and the cloth is impregnated with moisture. Impregnation with moisture reduces contact impedance. However, since the electrode in Comparative Example 2 needs to be impregnated with moisture to reduce the contact impedance, problems arise such as user discomfort and user inconvenience caused by presence of moisture in Comparative Example 2.

In contrast to Comparative Example 2, according to the present embodiment, since the conductive coating material 20 is formed inside the porous substrate 10, the contact impedance can be sufficiently reduced without the need for impregnation with moisture as in Comparative Example 2. As described above, since impregnation with moisture is unnecessary, the bioelectrode 100 can be used in a dry state. Accordingly, it is possible to reduce user discomfort caused by the presence of moisture and user inconvenience associated with the need for moisture impregnation.

In Comparative Example 3, an electrode is formed of a resin material in which conductive fillers are admixed. In Comparative Example 3, contact impedance is reduced as compared to Comparative Example 1. However, since it is difficult to ensure flexibility of the electrode of Comparative Example 3, reduction effect on the contact impedance is limited. According to the present embodiment, since the conductive coating material 20 is formed inside the porous substrate 10, contact impedance can be sufficiently reduced as compared with Comparative Example 3.

Since contact impedance is reduced as described above, in EMS devices or low-frequency therapeutic devices, an appropriate stimulus can be applied to a living body. Specifically, for example, it is possible to reduce a probability of a sharp tingling pain being generated in a living body, or a probability that a stimulus imparted to a living body is unable to be perceived. As a result of the reduced contact impedance, biological information can be measured with high accuracy in biometric devices.

Further, the inner surfaces of the plurality of pores 13 in addition to the surface of the porous substrate 10 are coated with the conductive coating material 20. According to the above-described configuration, the contact area between the porous substrate 10 and the coating material 20 is sufficiently secured as compared with a configuration in which the coating material 20 is formed only on the first surface 11 and the second surface 12 of the porous substrate 10. Therefore, even in a state in which the porous substrate 10 deforms (for example, expands or contracts), defects such as breakage or peeling of the coating material 20 are unlikely to occur. The conductivity of the bioelectrode 100 can be easily maintained as compared with a configuration in which an electrode is formed only on the surface of a flat substrate, for example. As described above, according to the present embodiment, both flexibility and conductivity of the bioelectrode 100 can be achieved.

Further, in the present embodiment, the inner surfaces of the plurality of pores 13 communicating between the first surface 11 and the second surface 12 of the porous substrate 10 are coated with the conductive coating material 20. Therefore, conductivity between the first surface 11 and the second surface 12 is easily ensured.

### B: Method for Manufacturing Bioelectrode 100

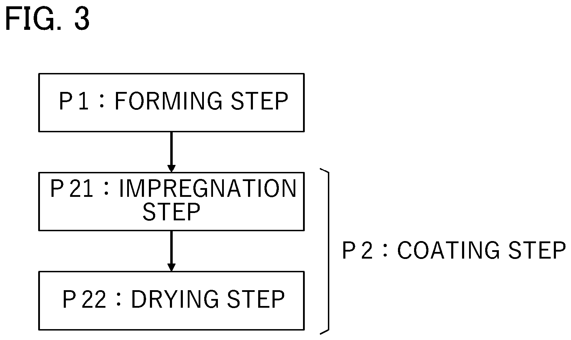

Fig. 3 is a flowchart illustrating an example of a manufacturing method of the bioelectrode 100 described above.

First, in a forming step P1, the porous substrate 10 having the plurality of pores 13 formed inside is formed. Specifically, a resin material mixed with a foaming agent is formed into a flat plate shape under heating while in a foamed state. As described above, various conditions concerning the forming step P1 are selected so that a continuous pore structure is formed in the porous substrate 10. The method of forming the porous substrate 10 is not limited to the above example.

In the coating step P2 performed after the forming step P1, the conductive coating material 20 is formed that coats the porous substrate 10. As described above, the coating material 20 is formed so as to coat the first surface 11 and the second surface 12 of the porous substrate 10, and the inner surfaces of the plurality of pores 13. As also described above, in the present embodiment, the conductive polymer is not added when forming the porous substrate 10 (forming step P1). The coating material 20 of the conductive polymer is formed after the porous substrate 10 is formed. The coating step P2 of the present embodiment includes an impregnation step P21 and a drying step P22.

In the impregnation step P21, the porous substrate 10 is impregnated with a coating solution comprising a conductive polymer (PEDOT). The coating solution is, for example, a solution in which a mixture of PEDOT and PSS is dispersed in a solvent. The solvent of the coating solution is, for example, pure water or an organic solvent (e.g., ethanol). For example, the coating solution penetrates the plurality of pores 13 inside the porous substrate 10 by discharging air in a working space to the outside (evacuation) while the entire porous substrate 10 is immersed in the coating solution.

In the drying step P22, the coating material 20 is formed by drying the porous substrate 10 impregnated with the coating solution. That is, for example, as a result of volatilization of the solvent of the coating solution under heating, a thin-film coating material 20 is formed that coats the first surface 11 and the second surface 12 of the porous substrate 10 and the inner surfaces of the plurality of pores 13.

According to the above-described manufacturing method, it is possible to manufacture the bioelectrode 100 capable of achieving both flexibility and conductivity as described above. In the present embodiment, in particular, the coating material 20 is formed by impregnating the porous substrate 10 after the substrate is formed, with the coating solution containing the conductive polymer; and volatilizing the solvent of the coating solution by drying the porous substrate 10. Therefore, the bioelectrode 100 can be manufactured by use of a simple process.

### C: Modifications

Specific examples of modifications of the above-described embodiments are described below. Two or more aspects freely selected from the following examples may be combined as appropriate as long as they do not contradict each other.

(1) In the above-described embodiment, the plurality of pores 13 is uniformly distributed over the entire area of the porous substrate 10. However, the plurality of pores 13 need not be evenly distributed. For example, the distribution density of the plurality of pores 13 may differ between a portion close to the first surface 11 and a portion close to the second surface 12 in a direction of the plate thickness of the porous substrate 10. In addition, for example, in a plane along the surface (the first surface 11, the second surface 12) of the porous substrate 10, the distribution density of the plurality of pores 13 may differ between a portion close to a peripheral edge of the bioelectrode 100 and a portion close to the center.
(2) In the above-described embodiment, the plurality of pores 13 formed in the porous substrate 10 have the same diameter. However, the plurality of pores 13 in the porous substrate 10 may be formed to have different diameters.
(3) In the above-described embodiment, the porous substrate 10 is formed of a single layer, but the porous substrate 10 may be formed by laminating a plurality of layers of different materials, with the plurality of pores 13 formed to be in communication with each other through the plurality of layers. In addition, in the above-described embodiment, the coating material 20 is formed of a single layer. However, the coating material 20 may be formed by laminating a plurality of layers of different materials.
(4) In the above embodiment, the porous substrate 10 is formed to have a flat plate shape. However, the shape of the porous substrate 10 may be freely selected and is not limited to the above example. The porous substrate 10 may be formed to have a three-dimensional shape such as a band shape that can be wound around a body part of a living body, such as a wrist or an ankle, for example.
(5) In the above embodiment, the porous substrate 10 is an insulator. However, the porous substrate 10 may be a conductor. For example, the porous substrate 10 may be formed from a resin material in which conductive materials such as conductive fillers are dispersed, or may be formed from various conductive polymers.

It is of note that, as described above, the resin material in which conductive fillers are admixed tends to have low flexibility. According to the bioelectrode 100 of the present disclosure, the conductivity of the bioelectrode 100 is ensured by the forming of the coating material 20. Therefore, even in a case in which the conductive fillers are dispersed in the porous substrate 10, an amount of the conductive fillers required to secure sufficient conductivity is reduced as compared with a conventional mode in which the coating material 20 is not formed. That is, insufficient flexibility of the porous substrate 10 caused by the conductive fillers is alleviated. Moreover, in the present disclosure, the plurality of pores 13 also contributes to the flexibility of the porous substrate 10. Therefore, even in a configuration in which the porous substrate 10 contains conductive fillers, the above-described effect of achieving both flexibility and conductivity of the bioelectrode 100 can be realized as compared with a configuration in which the coating material 20 or the plurality of pores 13 are not formed.

(6) The use of the bioelectrode 100 is freely selected. That is, the scope of the present disclosure is not limited to EMS devices, low-frequency therapeutic devices, or biometric devices described in the foregoing embodiment.

### D: Appendix

For example, the following configuration can be understood from the embodiments exemplified above.

A bioelectrode according to an aspect (Aspect 1) of the present disclosure includes a porous substrate having a plurality of pores formed inside; and a conductive coating material coating a surface of the porous substrate and inner surfaces of the plurality of pores. In the above aspect, since the porous substrate having a plurality of pores formed inside is used, flexibility is easily ensured as compared with, for example, a bioelectrode of a resin material admixed with conductive fillers. Therefore, contact impedance between the bioelectrode and the biological surface can be reduced. Further, since the inner surfaces of the plurality of pores in addition to the surface of the porous substrate is coated with a conductive coating material, defects such as breakage or peeling of the coating material are unlikely to occur even in a state where the porous substrate deforms. Therefore, it is easy to maintain conductivity as compared with a configuration in which an electrode is formed on the surface of a substrate. As described above, according to the present disclosure, both flexibility and conductivity of the bioelectrode can be achieved.

In an example (Aspect 2) of Aspect 1, the surface of the porous substrate includes a first surface and a second surface that oppose each other, and the porous substrate has a continuous pore structure in which two or more pores of the plurality of pores extend through an entire range from the first surface to the second surface. In the above aspect, the inner surfaces of the two or more pores communicating between the first surface and the second surface are coated with a conductive coating material. Therefore, it is easy to ensure conductivity between the first surface and the second surface.

In an example (Aspect 3) of Aspect 2, the coating material includes: a first portion coating the first surface; a second portion coating the second surface; and a third portion coating inner surfaces of the plurality of pores, and the first portion and the second portion are electrically connected to each other via the third portion. According to the above aspect, an electric current can flow through the first portion and the second portion.

In an example (Aspect 4) of any of Aspects 1 to 3, the coating is formed from a conductive polymer. In the above aspect, the coating material is formed from a conductive polymer. Therefore, the coating material can be easily formed on the inner surfaces of the plurality of pores in the porous substrate. In an example (Aspect 5) of Aspect 4, the conductive polymer contains PEDOT, for example.

In an example (Aspect 6) of any of Aspects 1 to 5, the coating material has a film thickness that is less than a radius of each of the plurality of pores. In the above aspect, a space is formed inside the coating material coating the inner surface of each pore. That is, the coating material in each pore is formed in a spherical shell shape. Therefore, sufficient flexibility of the bioelectrode is easily ensured as compared with a configuration in which the coating material is filled in the whole of each pore.

A method for manufacturing a bioelectrode according to an aspect (Aspect 7) of the present disclosure includes a forming step of forming a porous substrate having a plurality of pores formed inside; and a coating step of coating a surface of the porous substrate and inner surfaces of the plurality of pores with a conductive coating material. In the above aspect, since a porous substrate having a plurality of pores formed inside is used, sufficient flexibility is easily ensured as compared with, for example, a bioelectrode of a resin material admixed with conductive fillers. Therefore, the contact impedance between the bioelectrode and the surface of living body can be reduced. Further, since the inner surfaces of the plurality of pores in addition to the surfaces of the porous substrate are coated with a conductive coating material, defects such as breakage or peeling of the coating material are unlikely to occur even in a state where the porous substrate deforms. Therefore, it is easy to maintain the conductivity as compared with a configuration in which an electrode is formed on the surface of a substrate. As described above, according to the present disclosure, both flexibility and conductivity of the bioelectrode can be achieved.

In an example (Aspect 8) of Aspect 7, the coating step includes: an impregnation step of impregnating the porous substrate with a solution containing a conductive polymer; and a drying step of drying the porous substrate impregnated with the solution, to form the coating material. In the above aspect, the coating material is formed on the porous substrate after the porous substrate is formed, by impregnating the porous substrate with a solution containing the conductive polymer and volatilizing the solvent by drying the substrate. Therefore, the bioelectrode can be manufactured by use of a simple process.

### Description of Reference Signs

100... bioelectrode, 10... porous substrate, 11... first surface, 12... second surface, 13... pores, 20... coating material.

## Claims

1. A bioelectrode comprising:
a porous substrate having a plurality of pores formed inside; and
a conductive coating material coating a surface of the porous substrate and inner surfaces of the plurality of pores.

2. The bioelectrode according to claim 1, wherein:
the surface of the porous substrate includes a first surface and a second surface that oppose each other, and
the porous substrate has a continuous pore structure in which two or more pores of the plurality of pores extend through an entire range from the first surface to the second surface.

3. The bioelectrode according to claim 2, wherein:
the coating material includes:
a first portion coating the first surface;
a second portion coating the second surface; and
a third portion coating inner surfaces of the plurality of pores, and
the first portion and the second portion are electrically connected to each other via the third portion.

4. The bioelectrode according to any one of claims 1 to 3, wherein the coating material is formed from a conductive polymer.

5. The bioelectrode according to claim 4, wherein the conductive polymer contains PEDOT.

6. The bioelectrode according to any one of claims 1 to 5, wherein the coating material has a film thickness that is less than a radius of each of the plurality of pores.

7. A method for manufacturing a bioelectrode, the method comprising:
a forming step of forming a porous substrate having a plurality of pores formed inside; and
a coating step of coating a surface of the porous substrate and inner surfaces of the plurality of pores with a conductive coating material.

8. The method for manufacturing the bioelectrode according to claim 7,
wherein the coating step includes:
an impregnation step of impregnating the porous substrate with a solution containing a conductive polymer; and
a drying step of drying the porous substrate impregnated with the solution, to form the coating material.
